# EUROPEAN PATENT SPECIFICATION

(11) **EP 3 334 496 B1**
(45) Date of publication and mention of the grant of the patent: **30.06.2021**
(21) Application number: 16836027.9
(22) Date of filing: 15.08.2016
(51) Int. Cl.: A61N 5/10, G06T 7/00, G06F 3/0482, G16H 50/20, G06F 3/0484, G16H 20/40, G16H 30/40

(54) **RESULT-DRIVEN RADIATION THERAPY TREATMENT PLANNING**
ERGEBNISGESTEUERTE PLANUNG EINER STRAHLENTHERAPIEBEHANDLUNG
PLANIFICATION DE TRAITEMENT DE RADIOTHÉRAPIE PILOTÉE PAR LES RÉSULTATS

(30) Priority: 13.08.2015 US 201562204470 P
(43) Date of publication of application: 20.06.2018
(73) Proprietor: Siris Medical, Inc., Redwood City, CA 94063 (US)
(72) Inventor: CARPENTER, Colin, Morehouse, Redwood City, CA 94063 (US); PATTISON, Adam, J., Redwood City, CA 94063 (US)
(74) Representative: CSY London
(86) International application number: PCT/US2016/047041
(87) International publication number: WO 2017/027879

(56) References cited:
- US-A1- 2006 293 583
- US-A1- 2011 153 547
- US-A1- 2013 272 593
- US-A1- 2014 205 167
- US-A1- 2014 275 700
- US-A1- 2014 378 737

## Description

This invention relates generally to radiation treatment planning, and more specifically to planning relating to toxicity outcomes or treatment efficacy.

The process of determining the optimal therapy for patients afflicted with cancer has become an increasingly complex task, due to the overwhelming degrees of freedom and constraint priorities, which often change depending on the clinical care team, attending physicians, physicists, and available technology, and the uniqueness of the disease. Prospective clinical trials tend to have limited outcome scope and specificity, and homogenize a population. Complicating this is the variability in therapy such as radiation therapy, where the dosimetrist who creates a radiation plan has a difficult challenge in creating the optimal plan; this process is further challenged because the physicians and physicists are sporadically connected to the treatment planning process because of other demands on their time. In addition, the clinical care team does not know the precise probabilities of adverse events (including likely toxicity to anatomical structures and treatment efficacy) resulting from treatment during the treatment planning process, as they instead rely on data from studies with homogenized patient populations. Thus, the members of the clinical care team are limited in their abilities to make informed decisions about certain tactical trade-offs in a patient's treatment.
US 2006/293583 discloses a method of automatically optimizing an inverse treatment plan by referencing data from accepted plan libraries. In the disclosed method, a treatment planning optimizer automatically modifies one or more of the optimization constraints so that the planning result is guided towards one or more treatment plans in the plan library. Dose volume histograms of a current patient are compared with dose volume histograms of prior patients to determine whether the treatment plan outlined for the current patient is a previously accepted treatment plan.
US 2011/153547 discloses a method of determining a new treatment plan for a new patient. The method comprises providing a representation of the new patient's organ at risk relative to a target, searching for a prior treatment plan for a prior patient with a similar representation, and reviewing the prior treatment plan for the prior patient in order to determine whether the new treatment plan can be improved based on information in the prior treatment plan.
US 2014/378737 discloses a system for facilitating creation of a patient treatment plan. The system includes components configured to receive at least one feature associated with patient data and search a database of previously planned radiation treatments to identify one or more matching plans from the database based on the at least one feature. Parameters corresponding to the identified matching treatment plans may be used to facilitate creation of the patient treatment plan.
According to one aspect of the present invention there is provided a computer implemented method for creating a radiation treatment plan for a given patient as defined in claim 1.
According to another aspect of the present invention there is provided a computer system for creating a radiation treatment plan for a given patient as defined in claim 3.
Preferred features of the invention are recited in the dependent claims.

In embodiments of the present invention, a treatment planning engine informs a patient's clinical care team of the probabilities of adverse events as results of treatment for the patient during the process of planning the patient's treatment. By providing real-time information as to the likely therapeutic response of the disease and toxicity of the treatment to anatomical structures of the patient and as to the efficacy of the treatment for the patient, the engine allows the clinical care team to make informed decisions about tactical trade-offs in the patient's treatment. A preliminary stage in radiation treatment planning for a given patient involves a clinician defining the contours of the tumor volume and anatomical structures located near the tumor volume in patient images. The treatment planning engine described herein empowers radiation treatment decision makers, such as a clinician, to efficiently identify an optimal radiation treatment result for a given patient.

In operation, the treatment planning engine imports data for a given patient, such as patient images and patient contours that may have been previously defined by a clinician. Based in part on the defined contours, the treatment planning engine generates a set of recommended treatment results (e.g., toxicity to anatomical structure near to the tumor, efficacy of the treatment, including probability of tumor recurrence, period of time until recurrence, etc.) comprised of a treatment result prediction and a plurality of treatment result matches. The treatment result prediction is generated using a prediction model based on prior patient data including dose plans, medical history, and treatment results. The plurality of treatment result matches is identified from treatment results resulting from previously-administered dose plans that may be effective for the given patient. The treatment planning engine presents the set of recommended treatment results to the clinician for evaluation.

A user interface simultaneously displays the set of recommended treatment results with the patient images and contours on which the set of recommended treatment results are at least in part based. The clinician is able to adjust the contours in the user interface and, in real-time, evaluate the impact on the tumor volume and the toxicity risk to the nearby anatomical structures. With the user interface, the clinician can visualize the relationship between radiation delivery to the contours and the resulting recommended treatment results, allowing the clinician to make informed decisions about certain trade-offs during radiation treatment planning for the patient. The clinician may go through a cycle of modifying contours and optimizing a recommended treatment result, repeating the steps in any order and as many times as desired, until an optimal treatment result is selected for the patient.

### BRIEF DESCRIPTION OF THE DRAWINGS

Figure (or "FIG.") 1 illustrates a block diagram of a system environment for radiation treatment planning and delivery, according to an embodiment.
FIG. 2 illustrates a block diagram of a system for determining the recommended dose plans that are output by the dose prediction module, according to an embodiment.
FIG. 3 illustrates a system environment for generating a radiation dose plan prediction model, according to an embodiment.
FIG. 4 illustrates a block diagram of a system for determining recommended treatment results that are output by the treatment results module, according to an embodiment.
FIG. 5 illustrates a system environment for generating a treatment results prediction model, according to an embodiment.
FIG. 6 illustrates a system for optimizing a selected recommended treatment result, according to an embodiment.
FIG. 7 illustrates an exemplary user interface for visualizing recommended treatment results, according to an embodiment.
FIG. 8 illustrates an exemplary user interface for visualizing recommended treatment results, according to an additional embodiment.
FIG. 9 illustrates a flow diagram illustrating the steps for clinician-directed radiation treatment planning, according to an embodiment.

The figures depict various embodiments of the present invention for purposes of illustration only. The invention is defined in the following claims; other embodiments, aspects, methods etc. do not constitute a part of the invention.

### DETAILED DESCRIPTION

### System Architecture

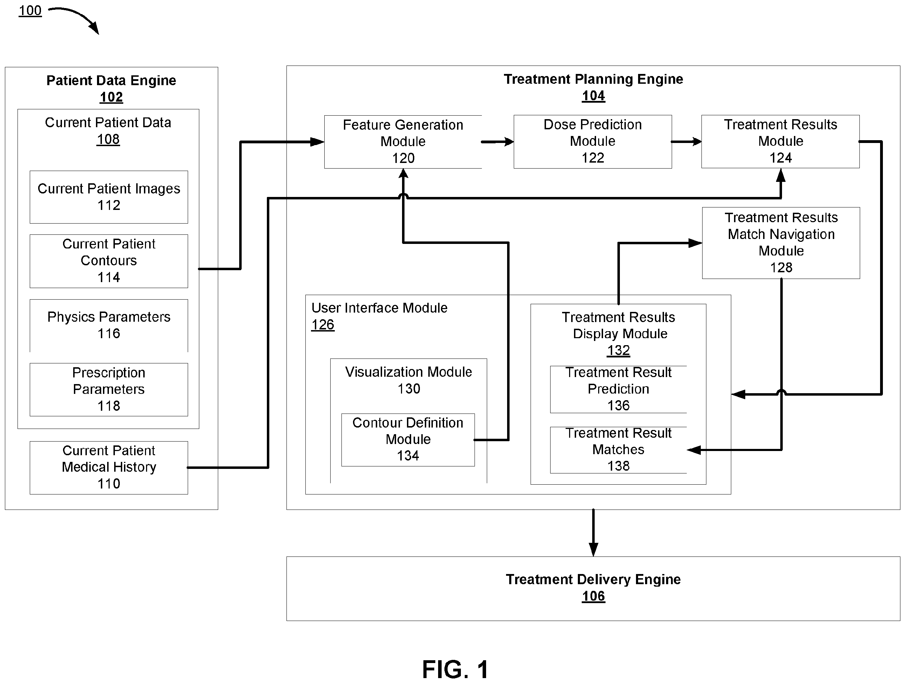

FIG. 1 illustrates a block diagram of a system environment 100 for radiation treatment planning and delivery, according to an embodiment. Some embodiments of the system environment 100 have different modules than those described here. Similarly, the functions can be distributed among the modules in a different manner than is described here. In certain embodiments of the system 100, a "treatment result" may be referred to as a "treatment outcome." Both phrases indicate the results of delivering a radiation therapy treatment plan (i.e., a "dose plan") to a patient. As shown, the system environment 100 includes a patient data engine 102, a treatment planning engine 104, and a treatment delivery engine 106.

The patient data engine 102 provides data associated with patients needing radiation treatment. The patient data may be imported into the patient data engine 102. In the embodiment of FIG. 1, data associated with a current patient includes current patient data 108 and current patient medical history 110.

The current patient data 108 is data associated with a current patient that is used to generate features associated with the current patient. The patient data engine 102 transmits the current patient data 108 to the treatment planning engine 104 to generate the features associated with the current patient. These generated features are related to a predicted radiation dose treatment plan in the treatment planning engine 104. These generated features can be compared to similar generated features of prior patients for the purpose of identifying one or more prior patients that are the most similar to the current patient. By matching these generated features, prior treatment results may be drawn from a database of previously-administered treatment plans to find a prior patient's treatment plan (i.e., dose plan) that is the closest match to providing an optimal treatment result for the current patient. The prior patient's treatment plan may inform the treatment planner or clinician of what may be expected when a similar treatment is delivered to the current patient. The generated features may also be used in prediction models to create dose predictions and treatment result predictions for a patient. In the embodiment of FIG. 1, current patient data 108 includes current patient images 112, current patient contours 114, physics parameters 116, and prescription parameters 118.

The current patient images 112 may include visual representations of the interior of a patient's body for medical purposes. These current patient images 112 may be produced by a medical imaging technology, such as computed tomography (CT), magnetic resonance imaging (MRI), X-ray, fluoroscopy, ultrasound, nuclear medicine including positron emission tomography (PET), or any other suitable medical imaging technology. In the embodiment of FIG. 1, the current patient images 112 may be a plurality of images, wherein each image illustrates the same perpendicular cross-section of the interior of the patient's body but at various depths of the patient's body. The current patient images 112 may have associated patient image parameters, such as distance, volume, geometric relationship, importance of structures and surrounding structures, and/or the like. The current patient images 112 and the associated patient image parameters may be imported into the patient data engine 102.

The current patient contours 114 are a set of contours identifying the three-dimensional tumor volumes and the anatomical structures located in the surrounding region of the tumor volumes that are captured in the current patient images 112. The contours identifying the three-dimensional tumor volumes indicate the targeted area of radiation dose delivery, while the contours identifying the surrounding anatomical structures indicate structures that may be at risk from the radiation dose delivery. Prior to importing the current patient data 108 into the patient data engine 102, the current patient contours 114 may be defined in each current patient image 112 by a clinician. In some embodiments, the current patient contours 114 may be automatically generated for the current patient images 112 using contouring techniques known in the field. The current patient contours 114 may be automatically generated using historical contours created for the current patient or a different patient having similar imaging data.

The physics parameters 116 are parameters of radiation delivery. Physics parameters may include penumbra, aperture, incident angle, beam energy, radiation type, depth of structure, existence of bolus, resolution of collimator, dose delivery rate, type of Treatment Planning System used, and other radiation delivery descriptive data. In some embodiments, the physics parameters 116 may not be included as inputs into the treatment planning engine 104.

The prescription parameters 118 are parameters regarding the method of radiation delivery. Prescription parameters may include fractionation schedule, treatment margin, number of beams or arcs, interpretation of contours, the clinicians involved in the treatment planning, and/or the like. In some embodiments, the prescription parameters 118 may not be included as inputs into the treatment planning engine 104.

In some embodiments, the current patient data 108 may further include disease parameters for the patient, such as disease stage, prior or post treatment therapy, prior radiation therapy, prior radiation damage to nearby tissue, disease type, disease histology, extent of the disease, prior disease, and/or the like.

The current patient medical history 110 is the medical history of a current patient that is used to improve the precision of the recommended treatment results. The current patient medical history 110 may include available therapeutic options, especially considering ability to pay for available therapies, genetic results, digital genetics, in vitro lab results, prior treatment history, comorbidities, etc. Examples of comorbidities include, but are not limited to, smoking history, cardiac or lung function, kidney function, diabetes, etc. Knowledge of a patient's medical history better informs the clinician of expected treatment results. For example, a patient who is a smoker is expected to have worse treatment results for lung function compared to a similar patient who is a non-smoker.

The treatment planning engine 104 processes patient data associated with a patient and recommends different treatment results to the patient's clinician, thus enabling the clinician to efficiently identify the optimal treatment result for the patient. Some embodiments of the treatment planning engine 104 have different modules than those described here. Similarly, the functions can be distributed among the modules in a different manner than is described here. The treatment planning engine 104 includes a feature generation module 120, a dose prediction module 122, a treatment results module 124, a user interface module 126, and a treatment result match navigation module 128.

The feature generation module 120 generates features associated with the current patient. In the embodiment of FIG. 1, the feature generation module 120 imports the current patient data 108 which includes current patient images 112, current patient contours 114, physics parameters 116, and prescription parameters 118. The feature generation module 120 uses the current patient data 108 to generate features associated with the current patient that can be compared to similar generated features associated with prior patients. These generated features can be used to identify, from a database, one or more prior patients that are most similar to the current patient. As previously described, by matching these generated features, prior treatment results may be drawn from a database of previously administered treatment plans to find a prior patient's treatment plan that is the closest match to providing an optimal treatment result for the current patient. The prior patient's treatment plan may inform the treatment planner or clinician of what may be expected when a similar treatment is delivered to the current patient.

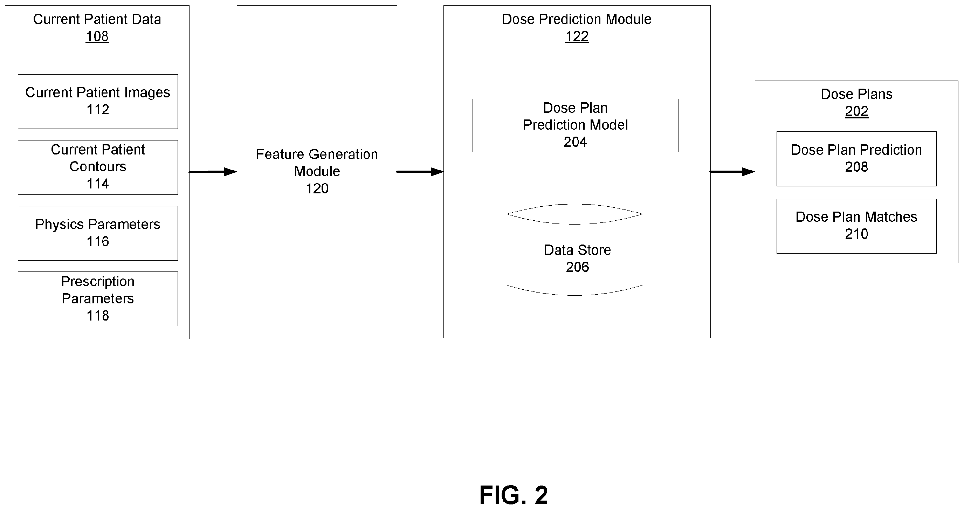

The dose prediction module 122 predicts the radiation dose delivered to tumor volumes and surrounding anatomical structures. In the embodiment of FIG. 1, the dose prediction module 122 outputs a set of recommended dose plans, which includes a plurality of dose plan matches and a dose plan prediction. The dose prediction module 122 generates the dose plan prediction for the current patient using a dose plan prediction model. The dose plan prediction model will be discussed in further detail with regards to FIGS. 2-3. Additionally, the dose prediction module 122 determines a plurality of previously-administered dose plans using the dose plan prediction of the current patient and the generated features associated with the current patient received from the feature generation module 120. The dose prediction module 122 compares the generated features of the current patient with the generated features of prior patients, as stored in a data store, to identify previously-administered dose plans within a threshold difference of the dose plan prediction. Those within the threshold difference are referred to as the dose plan matches. In the embodiment of FIG. 1, each dose plan match specifies the dose of radiation treatment previously-administered to one or more tumor volumes and surrounding anatomical structures. The plurality of dose plan matches and the dose plan prediction are input as a set of recommended dose plans into the treatment results module 124. In another embodiment, the clinician manually specifies the radiation dose to be delivered to the tumor volume without a prediction by the dose prediction module 122, and this is used as the dose plan prediction and is used to find dose plan matches with prior patients. This dose plan prediction and the dose plan matches are input as a set of recommended dose plans into the treatment results module 124.

The treatment results module 124 predicts the treatment results that may result from the recommended dose plans from the dose prediction module 122. As previously described, each dose plan specifies the dose of radiation treatment delivered to one or more tumor volumes and surrounding anatomical structures. As a result of delivering doses of radiation treatment to surrounding anatomical structures, damage may occur to these tissues, which may lead to side effects or symptoms. For example, tissue damage to the lungs may lead to pneumonitis (lung inflammation). The resulting tissue damage is defined as the "toxicity" to the anatomical structure. The level of toxicity is typically graded in severity (i.e., a "toxicity grade") and is measured by a healthcare provider during follow-up visits, performed in conjunction with the patient through self-assessment, and/or via lab tests.

In the embodiment of FIG. 1, a treatment result represents a set of one or more adverse events and a corresponding outcome for each adverse event that may result from a radiation treatment plan. Examples of an adverse event include myelitis, rib fracture, pericarditis, pneumonitis, or any other type of tissue damage. Each adverse event is associated with one or more of the following outcomes: a probability of level of toxicity to each identified anatomical structure, and a highest probable level of toxicity to each identified anatomical structure. The level of toxicity may be referred to as a "toxicity grade." As an example, the treatment results module 124 may predict an adverse event of pneumonitis that may have a 30% probability of being grade 3 pneumonitis (on a toxicity grade scale of 1-4, with 4 indicating the highest toxicity grade level). In various embodiments, the scale may vary.

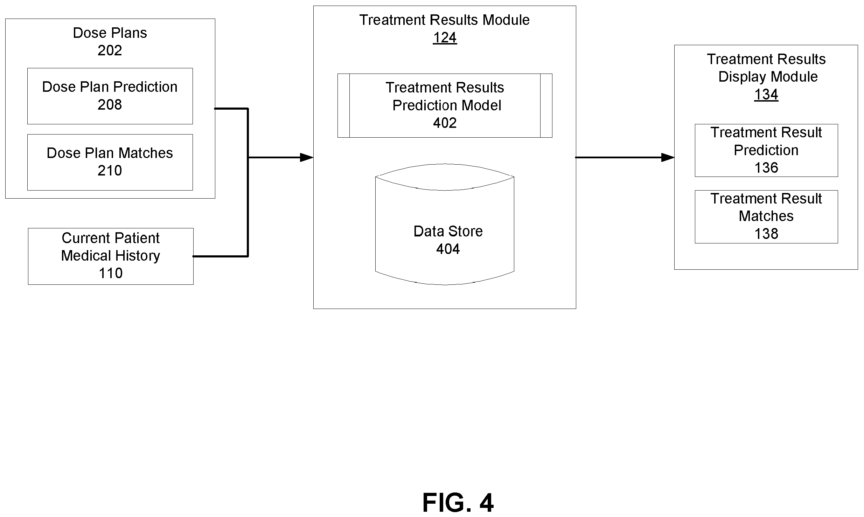

Similar to the dose prediction module 122, the treatment results module 124 outputs a set of recommended treatment results. In the embodiment of FIG. 1, the set of recommended treatment results includes a plurality of treatment result matches and a treatment result prediction. The treatment results module 124 generates the treatment result prediction for the current patient using a treatment results prediction model. The treatment results prediction model will be discussed in further detail with regards to FIGS. 4-5. Additionally, the treatment results module 124 determines a plurality of treatment results of previously-administered dose plans of prior patients using the treatment result prediction of the current patient and the set of recommended dose plans received from the dose prediction module 122. The treatment results module 124 compares the set of recommended dose plans with previously-administered dose plans of prior patients, as stored in a data store, to identify treatment results of previously administered dose plans of prior patients that are within a threshold difference of the treatment result prediction. Those within the threshold difference are referred to as the treatment result matches. The plurality of treatment result matches and the treatment result prediction are sent to the user interface module 126 for analysis by a clinician.

In some embodiments, the treatment results module 124 may factor in the current patient medical history 110 to improve the precision of the predicted treatment results. As previously described, the current patient medical history 110 may include available therapeutic options, especially considering ability to pay for available therapies, genetic results, digital genetics, in vitro lab results, prior treatment history, comorbidities, etc. For example, the treatment results module 124 may predict for a patient who smokes cigarettes an adverse event of pneumonitis with a 70% probability of grade 3 pneumonitis, rather than a 30% probability for a similar patient who doesn't smoke cigarettes. In some embodiments, a treatment result may also include one or more of the following: a probability of tumor recurrence, a type of tumor recurrence such as local recurrence or distant recurrence, and a probable length of time before tumor recurrence.

The user interface module 126 presents the recommended treatment results for analysis by a clinician. In the embodiment of FIG. 1, the user interface module 126 allows a clinician to visualize, interact with, and modify imaging data of the current patient and the set of recommended treatment results to achieve an optimal treatment result for the current patient. As illustrated in the embodiment of FIG. 1, the user interface module 126 includes a visualization module 130 and a treatment results display module 132.

The visualization module 130 displays imaging data of the current patient. In the embodiment of FIG. 1, the visualization module 130 imports the current patient images 112 and the associated current patient contours 114. As previously described, the current patient images 112 may be produced by a medical imaging technology, such as computed tomography (CT), magnetic resonance imaging (MRI), X-ray, fluoroscopy, ultrasound, nuclear medicine including positron emission tomography (PET), or any other suitable medical imaging technology. In the embodiment of FIG. 1, the visualization module 130 includes a contour definition module 134.

The contour definition module 134 is a contouring interface that allows a clinician to view and modify the current patient contours 114 as desired. As previously described, the current patient contours 114 are a set of contours that identify the three-dimensional tumor volumes and the anatomical structures located in the surrounding region of the tumor volumes that are captured in the current patient images 112. The contour definition module 134 overlays the plurality of contours onto the current patient images 112 for concurrent display in the visualization module 130. In the embodiment of FIG. 1, the contour definition module 134 is further configured to allow the clinician to add, delete, and/or modify the current patient contours 114. As a result, the contour definition module 134 creates a new set of current patient contours 114 that includes the added contours, the modified contours, and any remaining un-modified contours that haven't been deleted. The visualization module 130 is synced with the treatment results display module 132 such that modification of the contours in the visualization module 130 may cause the set of recommended treatment results to update in the treatment results display module 132.

The treatment results display module 132 displays the set of recommended treatment results that are determined by the treatment results module 124. The treatment results display module 132 is initially populated by the treatment results module 124 and allows a clinician to visualize and compare the recommended treatment results. In the embodiment of FIG. 1, the set of recommended treatment results displayed in the treatment results display module 132 are based on the current patient images 112 and the current patient contours 114 that are simultaneously displayed in the visualization module 130. The treatment results display module 132 includes a first portion that is dedicated to displaying the treatment result prediction 136 and a second portion that is dedicated to displaying the treatment result matches 138. The clinician may analyze each of the recommended treatment results to determine if any of the options are optimal treatment results for the current patient. In the embodiment of FIG. 1, the treatment results display module 132 allows the clinician to select one of the displayed recommended treatment results for modification or further optimization.

By simultaneously displaying the visualization module and the treatment results display module 132, a clinician may visualize the relationship between radiation delivery to the current patient contours 114 and the resulting recommended treatment results. The clinician is able to adjust contours and in real-time evaluate the impact of the tumor treatment and the toxicity risk to the nearby anatomical structures. This configuration allows the clinician to make informed decisions about certain trade-offs during radiation treatment planning for the current patient. In the embodiment of FIG. 1, the clinician may interact with the visualization module 130 to modify the current patient contours 114, or the clinician may interact with the treatment results display module 132 to select a recommended treatment result for optimization, allowing the clinician to create a treatment plan with optimal treatment results.

In the event that the clinician modifies the current patient contours 114 using the contour definition module 134 of the visualization module 130, the contour definition module 134 creates a new set of current patient contours 114 that includes the added contours, the modified contours, and any remaining un-modified contours that haven't been deleted. In the embodiment of FIG. 1, the new set of current patient contours 114 is sent to the feature generation module 120. As a result, the feature generation module 120 generates a new set of features associated with the current patient based on the current patient images 112, the new set of current patient contours 114, the physics parameters 116, and the prescription parameters 118. Subsequently, the dose prediction module 122 generates a new set of recommended dose plans based on the new set of generated features, the treatment results module 124 generates a new set of recommended treatment results based on the new set of recommended dose plans and the current patient medical history 110, and the treatment results display module 132 is updated to display the new set of recommended treatment results. The clinician may analyze the new set of recommended treatment results to determine if any of the options are optimal treatment results for the current patient.

In the event that the clinician selects a recommended treatment result in the treatment results display module 132 for optimization, the selected recommended treatment result and a set of filtering criteria are sent to the treatment result match navigation module 128. In the embodiment of FIG. 1, the set of filtering criteria is defined by the clinician in the treatment results display module 132. The clinician may define the filtering criteria to exclude an adverse event from the treatment result or to place a threshold limit on an outcome of an adverse event. The treatment result match navigation module 128 uses the selected recommended treatment result and the set of filtering criteria to search through a data store of prior patient treatment results to identify a new set of treatment result matches for the current patient. Each of the new treatment result matches is associated with prior patients similar to the current patient. In the embodiment of FIG. 1, the treatment result match navigation module 128 sends the new set of treatment result matches to the treatment results matches 138 for display in the treatment results display module 132. The clinician may analyze the new set of recommended treatment results to determine if any of the options are optimal treatment results for the current patient. In the embodiment of FIG. 1, the clinician may go through a cycle of modifying contours and optimizing a selected recommended treatment result, repeating the steps in any order and as many times as desired, until an optimal treatment result is selected for the current patient.

The treatment delivery engine 106 enables a treatment planner to create a patient-specific treatment delivery plan based on the treatment result selected by the clinician in the treatment planning engine 104. In the embodiment of FIG. 1, the selected treatment result informs the treatment planner of the clinically appropriate profile of radiation to be delivered including but not limited to radiation intensity, angle of delivery, multi-leaf collimator status, temporal fractionation, anatomy, presentation of the tumor, anticipated treatment results, prior clinical staff, and anticipated treatment course.

Based on the treatment delivery plan, the treatment delivery engine 106 generates a patient-specific delivery template that configures a radiation therapy machine for delivering the radiation treatment to the patient. In one embodiment, the treatment delivery engine 106 interacts with a therapy machine control interface that is configured with standard communication protocols. The patient-specific delivery template identifies the tumor volumes as well as the anatomical structures that are to receive radiation treatment. For each volume or structure, the delivery template may also specify the percentage volume that is to receive radiation treatment and the dose of treatment to be delivered. In addition, this template may specify the optimization objects, treatment protocols, beam orientations, collimator/multi-leaf collimator positions, couch positions, and other parameters known in the art.

FIG. 2 illustrates a block diagram of a system for determining the recommended dose plans that are output by the dose prediction module 122, according to an embodiment. As previously described, a dose plan specifies the dose of radiation treatment delivered to one or more tumor volumes and surrounding anatomical structures. As illustrated in FIG. 2, the system includes the current patient data 108, the feature generation module 120, the dose prediction module 122, and dose plans 202.

As described with regards to FIG. 1, the current patient data 108 is data associated with a current patient that is used to generate features associated with the current patient. In the embodiment of FIG. 2, the current patient data 108 includes current patient images 112, current patient contours 114, physics parameters 116, and prescription parameters 118. The current patient data 108 is input into the feature generation module 120 to generate features associated with the current patient.

The dose prediction module 122 predicts the radiation dose delivered to tumor volumes and surrounding anatomical structures. As previously described, the dose prediction module 122 uses the generated features of the current patient received from the feature generation module 120 to determine a set of recommended dose plans. In the embodiment of FIG. 2, the dose prediction module 122 includes a dose plan prediction model 204 and a data store 206. The dose prediction module 122 outputs dose plans 202, which is a set of recommended dose plans including a dose plan prediction 208 and a plurality of dose plan matches 210.

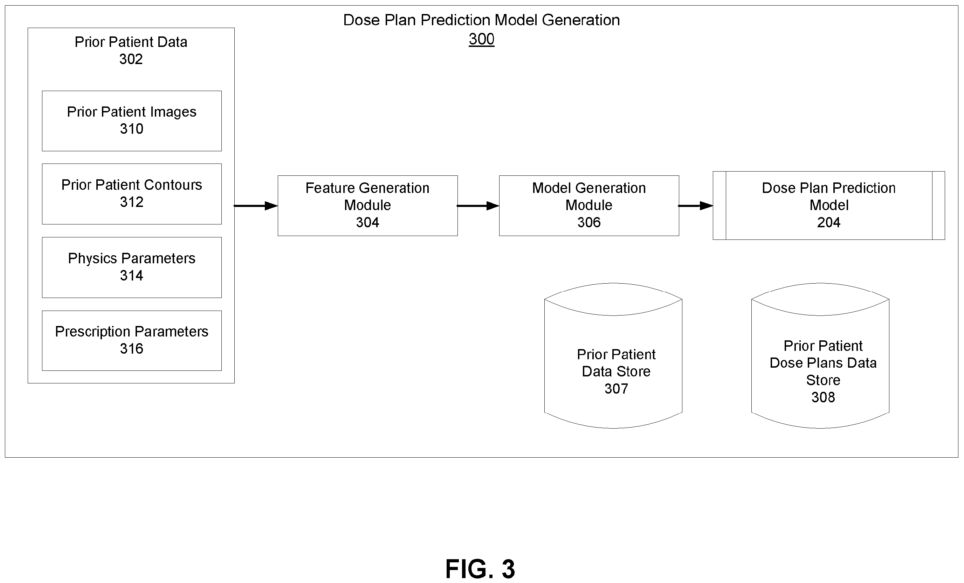

The dose plan prediction model 204 is a prediction model used to generate the dose plan prediction 208. In the embodiment of FIG. 2, the dose plan prediction model 204 is created using historical data of prior patients that have received radiation treatment. FIG. 3 illustrates a system environment 300 for generating a radiation dose plan prediction model, according to an embodiment. As illustrated, the dose plan prediction model generation system 300 includes prior patient data 302, a feature generation module 304, a model generation module 306, and a prior patient dose plans data store 308 to generate the dose plan prediction model 204.

The prior patient data 302 is data associated with a prior patient that is used to generate features associated with the prior patient. Similar to the current patient data 108, in the embodiment of FIG. 3, the prior patient data 302 includes prior patient images 310, prior patient contours 312, physics parameters 314, and prescription parameters 316. Similarly, the characteristics of the current patient images 112, the current patient contours 114, the physics parameters 116, and the prescription parameters 118 may be incorporated herein for the prior patient images 310, the prior patient contours 312, the physics parameters 314, and the prescription parameters 316, respectively. The prior patient data 302 may be historical data gathered from databases from a plurality of hospitals, clinics, cancer treatment centers, or any other center for radiation therapy. In addition to gathering the prior patient data 302, historical data regarding dose plans for each prior patient is gathered as well. In the embodiment of FIG. 3, the prior patient data 302, once gathered from other databases, may be stored in the prior patient data store 307. The prior patient dose plans may be stored the prior patient dose plans data store 308 in three-dimensional point sets or in dose volume histograms (DVHs).

The feature generation module 304 generates features associated with a prior patient. In the embodiment of FIG. 3, the feature generation module 304 imports the prior patient data 302, which includes prior patient images 310, prior patient contours 312, physics parameters 314, and prescription parameters 316. For a plurality of the prior patients stored in the prior patient data store 307, the feature generation module 304 generates a set of features associated with a specific prior patient using the prior patient's respective prior patients feature data 302. Similar to the generated features of the current patient as described with regards to FIG. 1, these generated features are related to a predicted radiation dose treatment plan. For each prior patient, the set of generated features may be stored in the prior patient data store 307. In some embodiments, the feature generation module 304 is functionally the same as the feature generation module 120.

The model generation module 306 generates the dose plan prediction model 204 by using the stored data of each prior patient in the prior patient data store 307 and the prior patient dose plans data store 308. In the embodiment of FIG. 3, the model generation module 306 accesses the set of generated features and prior patient dose plans for each prior patient and performs a regression analysis to create the dose plan prediction model 204 that relates the generated features to the dose plans. The dose plan prediction model 204 may be saved within the dose prediction module 122 such that the dose plan prediction model 204 may be used innumerous times to generate dose plan predictions for a plurality of patients to receive radiation treatment. The dose plan prediction model 204 may also be updated through machine-learning techniques and as additional prior patient data 302 and associated dose plans are gathered from other databases.

Returning to FIG. 2, the dose plan prediction model 204 may be used to generate the dose plan prediction 208 for a current patient. The generated features of the current patient from the feature generation module 120 are used as inputs into the dose plan prediction model 204. Since the dose plan prediction 208 is generated using a prediction model, the dose plan prediction 208 may not be a previously-administered dose plan of a prior patient. In some embodiments, the dose plan prediction 208 generated by the dose plan prediction model 204 is stored as a historical data point within the data store 206 and may be used as an additional data point to update the dose plan prediction model 204.

The data store 206 is a data store of previously-administered dose plans of prior patients and generated features associated with prior patients. In some embodiments, the data store 206 may be the same data store as the prior patient data store 307, the prior patient dose plans data store 308, or the data store 206 may combine the data stored within the data stores 307 and 308. In other embodiments, the data store 206 may be created once the generated features of the current patient are input into the dose plan prediction model 204 and a set of dose plans of prior patients from the prior patient dose plans data store 308 may be identified that are similar to the resulting dose plan prediction 208.

In the embodiment of FIG. 2, the dose prediction module 122 accesses the data store 206 to retrieve dose plans and generated features associated with prior patients. The dose prediction module 122 performs a multi-feature comparative analysis between a) the generated features associated with the current patient and the dose plan prediction 208, and b) the generated features associated with prior patients to identify one or more previously-administered dose plans of the prior patients that are within a threshold difference of the dose plan prediction 208. The identified previously-administered dose plans may be the most suitable for the current patient, referred to as the dose plan matches 210. Alternatively, the dose prediction module 122 may identify a subset of similar prior patients based on the generated features, herein referred to as a "dose plan neighborhood." The dose plan neighborhood may beneficially inform the clinician of a variety of dose plans based on prior patient contours, radiation delivery technology, and/or different treatment therapies. In the embodiment of FIG. 2, once the dose plan prediction model 204 generates the dose plan prediction 208 and the dose prediction module 122 identifies the dose plan matches 210, the dose prediction module 122 outputs the dose plans 202 to the treatment results module 124.

FIG. 4 illustrates a block diagram of a system for determining recommended treatment results that are output by the treatment results module 124, according to an embodiment. As previously described, a treatment result of a treatment plan represents a set of one or more adverse events and a corresponding outcome for each adverse event that may result from radiation delivered to an anatomical structure. In the embodiment of FIG. 4, the system includes the dose plans 202, the current patient medical history 110, the treatment results module 124, and the treatment results display module 134.

As described with regards to FIG. 2, the dose plans 202 are a set of recommended dose plans including the dose plan prediction 208 and the plurality of dose plan matches 210. The dose plans 202 and the current patient medical history 110 are input into the treatment results module 124 to determine a set of recommended treatment results.

The treatment results module 124 predicts the treatment results that may result from the recommended dose plans that are determined by the dose prediction module 122. As previously described, the treatment results module 124 uses the dose plans 202 and the current patient medical history 110 to determine a set of recommended treatment results. In the embodiment of FIG. 4, the treatment results module 124 includes a treatment results prediction model 402 and a treatment results data store 206. The treatment results module 124 outputs the treatment result prediction 136 and the plurality of treatment result matches 138 for display in the treatment results display module 134.

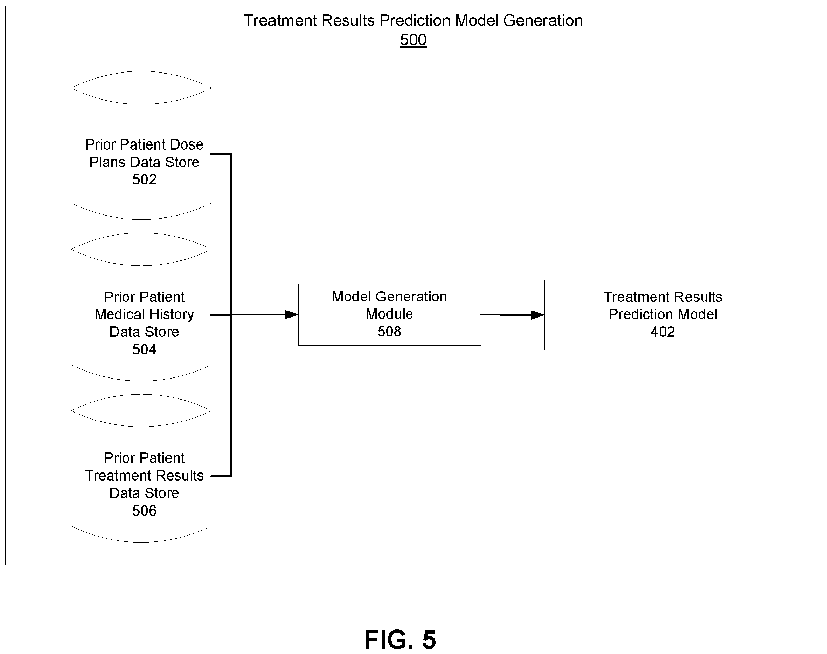

The treatment results prediction model 402 is a prediction model used to generate the treatment result prediction 136. In the embodiment of FIG. 4, the treatment results prediction model is created using historical data of prior patients that have received radiation treatment. FIG. 5 illustrates a system environment 500 for generating a treatment results prediction model 402, according to an embodiment. As illustrated, the treatment results prediction model generation system 500 includes a prior patient dose plans data store 502, a prior patient medical history data store 504, a prior patient treatment results data store 506, and a model generation module 508 to generate the treatment results prediction model 402.

The prior patient dose plans data store 502 is a data store of previously-administered dose plans of prior patients. In some embodiments, the prior patient dose plans data store 502 may be the same data store as the prior patient dose plans data store 308. The prior patient medical history data store 504 is a data store of medical history of prior patients. As previously described, medical history may include available therapeutic options, especially considering ability to pay for available therapies, genetic results, digital genetics, in vitro lab results, prior treatment history, comorbidities, etc. The prior patient treatment results data store 506 is a data store of treatment results of previously-administered dose plans of prior patients. The prior patient data stored in data stores 502, 504, and 506 may be historical data gathered from databases from a plurality of hospitals, clinics, cancer treatment centers, or any other center for radiation therapy. The prior patient dose plans may be stored in the prior patient dose plans data store 502 in three-dimensional point sets or in dose volume histograms (DVHs).

The model generation module 508 generates the treatment results prediction model 402 by using the stored data of each prior patient in the prior patient dose plans data store 502, the prior patient medical history data store 504, and the prior patient treatment results data store 506. In the embodiment of FIG. 5, the model generation module 508 accesses the dose plans, medical history, and treatment results for each prior patient and performs a regression analysis to create the treatment results prediction model 402 that relates the dose plans and medical history to the treatment results. The treatment results prediction model 402 may be saved within the treatment results prediction module 124 such that the treatment results prediction model 402 may be used innumerous times to generate treatment result predictions for a plurality of patients to receive radiation treatment. The treatment results prediction model 402 may also be updated through machine-learning techniques and as additional prior patient data is gathered from other databases.

Returning to FIG. 4, the treatment results prediction model 402 may be used to predict the treatment result prediction 136 for a current patient. The treatment results prediction model 402 receives the dose plans 202 and the current patient medical history 110 as inputs into the treatment results prediction model 402. Since the treatment result prediction 136 is generated using a prediction model, the treatment result prediction 136 may not be a treatment result of a previously-administered dose plan of a prior patient. In some embodiments, the treatment result prediction 136 generated by the treatment results prediction model 402 is stored as a historical data point within the data store 404 and may be used as an additional data point to update the treatment results prediction model 402.

The data store 404 is a data store of treatment results of previously-administered dose plans of prior patients, previously-administered dose plans of prior patients, and prior patient medical history. In some embodiments, the data store 404 may be the same data store as the prior patient dose plans data store 502, the prior patient medical history data store 504, or the prior patient treatment results data store 506, or the data store 404 may combine the data stored in the three data stores 502, 504, and 506. In the embodiment of FIG. 4, the treatment results module 124 accesses the data store 404 to retrieve treatment results, dose plans, and medical history associated with prior patients. Similar to the dose prediction module 122, the treatment results module 124 performs a multi-feature comparative analysis between a) the dose plans 202, the current patient medical history 110, and the treatment result prediction 136, and b) the previously-administered dose plans of prior patients and prior patient medical history to identify one or more treatment results of previously-administered dose plans of prior patients that are within a threshold difference of the treatment result prediction 136. Alternatively, the treatment results module 124 may identify a subset of similar prior patients based on the dose plans and medical history, herein referred to as a "treatment result neighborhood." The treatment result neighborhood may beneficially inform the clinician of a variety of treatment results based on prior patient contours, prior patient dose plans, radiation delivery technology, and/or different treatment therapies. The identified treatment results may be the most suitable for the current patient, referred to as the treatment result matches 138. In the embodiment of FIG. 4, once the treatment results prediction model 402 generates the treatment result prediction 136 and the treatment results module 124 identifies the treatment result matches 138, the treatment results module 124 outputs the set of recommended treatment results to the treatment results display module 134 for analysis by a clinician.

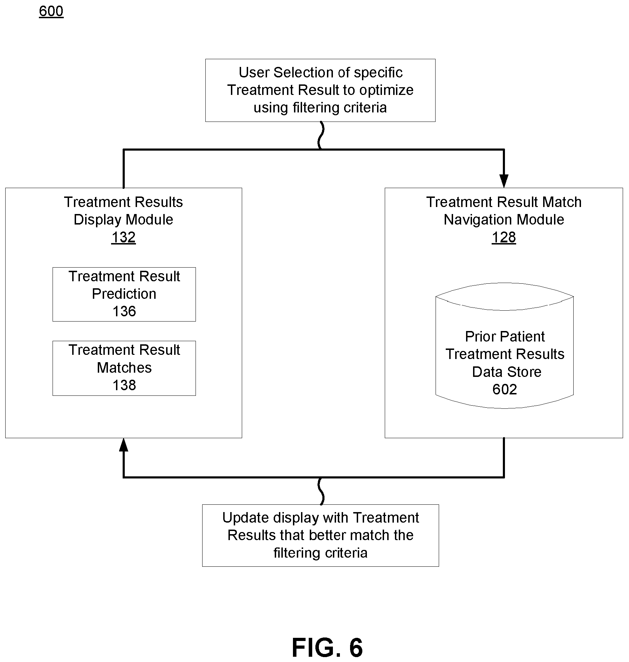

FIG. 6 illustrates a system 600 for optimizing a selected recommended treatment result, according to an embodiment. As illustrated in FIG. 6, the system 600 includes the treatment results display module 132 which includes the treatment result prediction 136 and the treatment result matches 138. The system 600 further includes the treatment result match navigation module 128 which includes a prior patient treatment results data store 602.

The treatment results display module 132 displays the set of recommended treatment results, comprised of the treatment result prediction 136 and the plurality of treatment result matches 138, that are determined by the treatment results module 124. The treatment results display module 132 allows a clinician to analyze each of the recommended treatment results to determine if any of the options are optimal treatment results for the current patient. In the embodiment of FIG. 6 the treatment results display module 132 allows the clinician to select one of the displayed recommended treatment results for modification or further optimization.

Upon selecting one of the displayed recommended treatment results, the clinician may define, within the treatment results display module 132, one or more filtering criteria with which to optimize the selected recommended treatment result. The clinician may define the filtering criteria to exclude an adverse event from the treatment result or to place a threshold limit on an outcome of an adverse event. For example, the clinician may specify a first filtering criteria to exclude bone fracture as an adverse event and a second filtering criteria to limit pneumonitis to a toxicity grade of level 1 (on a scale of 1-4, with 4 as the most severe). The filtering criteria are input into the treatment result match navigation module 128.

The treatment result match navigation module 128 uses the selected recommended treatment result and the set of filtering criteria to search through the prior patient treatment results data store 602 to identify a new set of treatment result matches 138 for the current patient. Each of the new treatment result matches is associated with prior patients similar to the current patient. The new set of treatment result matches 138 may provide recommended treatment results that are optimized based on the filtering criteria relative to the selected recommended treatment result. In the embodiment of FIG. 6, the treatment result match navigation module 128 sends the new set of treatment result matches to the treatment results matches 138 for display in the treatment results display module 132. The clinician may analyze the new set of recommended treatment results to determine if any of the options are optimal treatment results for the current patient. In the embodiment of FIG. 6, the clinician may go through a cycle of modifying contours and optimizing a selected recommended treatment result, repeating the steps in any order and as many times as desired, until an optimal treatment result is selected for the current patient.

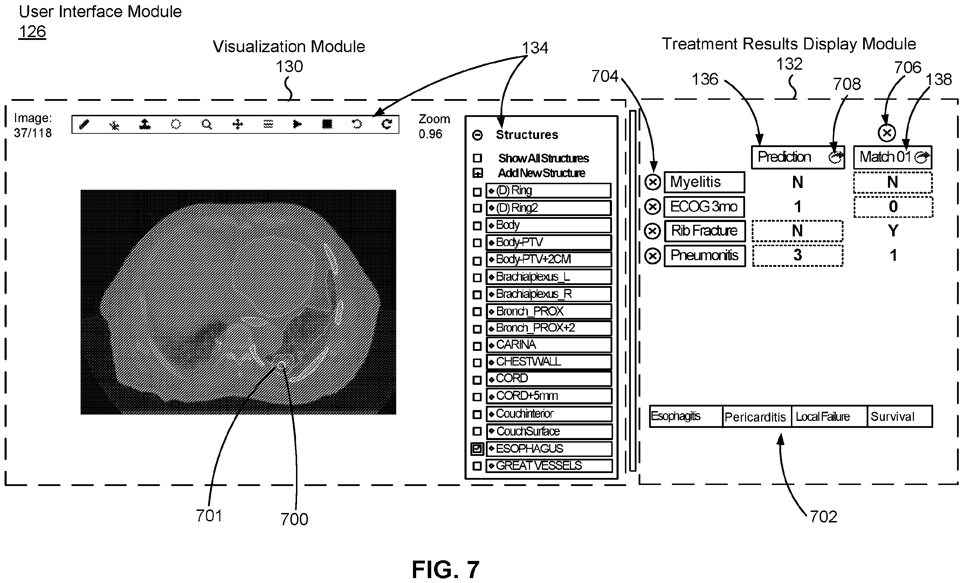

FIG. 7 illustrates an exemplary user interface for visualizing recommended treatment results, according to an embodiment. In the embodiment of FIG. 7, the user interface module 126 simultaneously displays the visualization module 130 with the treatment results display module 132. In the embodiment of FIG. 7, the user interface module 126 allows a clinician to visualize, interact with, and modify imaging data of the current patient and the set of recommended treatment results to achieve an optimal treatment result for the current patient.

The visualization module 130 displays imaging data of the current patient. In the embodiment of FIG. 7, the visualization module 130 imports the current patient images 112 and the corresponding current patient contours 114. As previously described, the current patient images 112 may be produced by a medical imaging technology, such as computed tomography (CT), magnetic resonance imaging (MRI), X-ray, fluoroscopy, ultrasound, nuclear medicine including positron emission tomography (PET), or any other suitable medical imaging technology. In the embodiment of FIG. 7, the current patient images 112 may be a plurality of images, wherein each image illustrates the same perpendicular cross-section of the interior of the patient's body but at various depths of the patient's body. The user interface module 126 may be configured to allow a clinician to scroll through the current patient images 112 such that cross-sections at various depths of the patient's body can be displayed in the visualization module 130 with the corresponding contours for each image. In the embodiment of FIG. 1, the visualization module 130 includes a contour definition module 134.

The contour definition module 134 is a contouring interface that allows a clinician to view and modify the current patient contours 114 as desired. As previously described, the current patient contours 114 are a set of contours that identify the three-dimensional tumor volumes and the anatomical structures located in the surrounding region of the tumor volumes that are captured in the current patient images 112. The contours surrounding the one or more tumor volumes indicate the areas towards which a radiation dose is targeted. The contours surrounding the one or more tumor volumes may be defined in view of standard practices relating to gross tumor volume (GTV), clinical tumor volume (CTV), and planning tumor volume (PTV), such that these contours may include portions of surrounding anatomical structures. The contours for the surrounding anatomical structures may highlight either a portion of or all of the anatomical structure. The contour definition module 134 overlays the plurality of contours onto the current patient images 112 for concurrent display in the visualization module 130. As illustrated in FIG. 7, imaging data of the current patient is shown with a plurality of current patient contours 114 in the visualization module 130. Each outline in the image is a contour line, and each contour line can be a different color to represent a different structure. The different colors can be indicated in a key to the right that lists the name of each structure in the current image in the visualization module 130. Using the key, the clinician can quickly identify which contour corresponds to which structure in the list. In the embodiment of FIG. 7, a contour 700 surrounds the gross tumor volume (GTV), a contour 701 surrounds the planning tumor volume (PTV), and the remaining contours highlight neighboring anatomical structures. In some images, various contours may overlap with each other, as shown in FIG. 7. In the embodiment of FIG. 7, the current patient contours 114 are imported with the current patient images 112 such that the contours readily appear with each image displayed in the visualization module 130. In some embodiments, the contour definition module 134 may be configured to automatically generate contours or may provide suggested contours for the current patient images 112.

In the embodiment of FIG. 7, the contour definition module 134 is configured to allow a clinician to add, delete, and/or modify the current patient contours 114. The contour definition module 134 includes a toolbar that allows the clinician to modify existing contours displayed in the visualization module 130. Using the toolbar, the clinician may change the shape of a selected contour. For example, a clinician may wish to modify an existing contour if a contour was initially defined incorrectly or to increase the margin of a contour surrounding a tumor volume. The contour definition module 134 also includes a structures list, which represents the various anatomical structures shown in the displayed current patient image 112. By selecting or de-selecting anatomical structures in the structures list, the contour definition module 134 causes contours for the anatomical structure to appear or disappear from the visualization module 130. The clinician may also add a new contour for an anatomical structure that may not have been initially defined. Adding and creating contours for an anatomical structure in the contour definition module 134 results in the treatment results display module 132 updating to include adverse events associated with the anatomical structure and the corresponding outcomes of the adverse events.

In the embodiment of FIG. 7, the contour definition module 134 is configured to detect if a contour has been added, deleted, and/or modified by a clinician. As a result, the contour definition module 134 captures the new set of current patient contours 114 that includes the added contours, the modified contours, and any remaining un-modified contours that haven't been deleted and sends the new set of current patient contours 114 to the feature generation module 120 so that a new set of recommended treatment results is subsequently determined. In the embodiment of FIG. 7, the visualization module 130 is synced with the treatment results display module 132 such that modification of the contours in the contour definition module 134 cause the set of recommended treatment results to update in the treatment results display module 132 in real-time. In some embodiments, the user interface module 126 may require the clinician to indicate when a new set of current patient contours 114 is ready to be captured, such that the treatment results display module 132 can be updated accordingly.

The treatment results display module 132 displays the set of recommended treatment results that are determined by the treatment results module 124. The treatment results display module 132 is initially populated by the treatment results module 124 and allows a clinician to visualize and compare the recommended treatment results. In the embodiment of FIG. 1, the set of recommended treatment results displayed in the treatment results display module 132 are based on the current patient images 112 and the current patient contours 114 that are simultaneously displayed in the visualization module 130. The clinician may analyze each of the recommended treatment results to determine if any of the options are optimal treatment results for the current patient.

In the embodiment of FIG. 7, the treatment results display module 132 presents the recommended treatment results in a table format. Each treatment result represents a set of one or more adverse events and a corresponding outcome for each adverse event that may result from a radiation treatment plan. As illustrated in FIG. 7, each column heading labels a recommended treatment result, while each row labels an adverse event associated with the current patient contours 114 in the visualization module 130. Examples of adverse events, as shown in FIG. 7, may include "Myelitis," "ECOG 3mo" (a physician's assessment of a patient's performance status), "Rib Fracture," and "Pneumonitis." The column labeled "Prediction" displays the treatment result prediction 136, listing the predicted outcome for each adverse event. The neighboring column labeled "Match 01" displays a first treatment result match 138, listing an outcome for each adverse event that resulted from a previously-administered treatment plan of a prior patient. The treatment results display module 132 may display multiple neighboring columns if there are multiple treatment result matches 138, e.g. "Match 02," "Match 03," etc. In some embodiments, the treatment results display module 132 may limit the number of treatment result matches 138 to avoid displaying less similar treatment result matches. By displaying the treatment result prediction 136 simultaneously with the treatment result matches 138, this configuration allows a clinician compare to the treatment result prediction 136 to the treatment result matches 138 to determine that the treatment result prediction 136 is a reasonable treatment result and that the clinician may accurately expect the treatment result as predicted, serving as a confidence check for the clinician.

In the embodiment of FIG. 7, each element of the table describes an outcome of an adverse event of a corresponding recommended treatment result. Each adverse event is associated with one or more of the following outcomes: a probability of level of toxicity to each identified anatomical structure, and a highest probable level of toxicity to each identified anatomical structure. The level of toxicity may be referred to as a "toxicity grade." In the embodiment of FIG. 7, for ease of display, only the highest probable toxicity grade may be displayed for each adverse event. For example, for the treatment result prediction 136, the adverse event "Pneumonitis" has an outcome of "3," indicating that toxicity grade 3 pneumonitis is more likely to occur than any other toxicity grade of pneumonitis. For some adverse events, the adverse event may have a binary outcome, such as "Yes, adverse event will occur," or "No, adverse event will not occur." For example, for the treatment result match 138, the adverse event "Rib Fracture" has an outcome of "Y," indicating that a rib fracture occurred as a result of the treatment plan for this prior patient. Other adverse events may be characterized by a level of function rather than a level of damage. For example, the adverse event "ECOG 3mo" is a physician's assessment of a patient's overall performance status at three months post-therapy. As shown in FIG. 7, for the treatment result prediction 136, "ECOG 3mo" has an outcome of "1," indicating functional level 1 (on a scale of 0-5, with 0 as the highest functional level). In various embodiments, the scale may vary. In some embodiments, the treatment results display module 132 may also display treatment efficacy for each recommended treatment result, such as a probability of tumor recurrence, a type of tumor recurrence such as local recurrence or distant recurrence, and a probable length of time before tumor recurrence. In the embodiment of FIG. 7, the outcomes may be emphasized in various colors or with various markings to indicate preferred outcomes when comparing outcomes of adverse events between recommended treatment results. As illustrated in FIG. 7, an outcome in a dashed box may be preferred over an outcome without a dashed box (e.g., "No rib fracture" is a preferable outcome over "Yes, rib fracture"). Various outcomes may also be emphasized with a specific color or marking to indicate an unacceptable outcome, which may be clinician-specific.

The treatment results display module 132 may include a variety of buttons that enable a clinician to modify the information shown in the treatment results display module 132. These modifications may allow the clinician to display only information that is deemed relevant or important to the current patient, facilitating an efficient, streamlined treatment planning process for the clinician. In the embodiment of FIG. 7, the buttons include a plurality of adverse event buttons 702, a plurality of adverse event removal buttons 704, a plurality of treatment result removal buttons 706, and a plurality of treatment result optimization buttons 708.

The plurality of adverse event buttons 702 represent additional adverse events that can be considered as a part of the recommended treatment results. In the embodiment of FIG. 7, the plurality of adverse event buttons 702 may be located below the table of recommended treatment results. A clinician may select one or more of the adverse event buttons if the clinician would like to determine the corresponding outcome of the adverse event for each recommended treatment result. When an adverse event button is selected, that adverse event is added as an additional row in the table of recommended treatment results, and the corresponding outcome of the adverse event for each recommended treatment result will populate the elements of the row. In some embodiments, the plurality of adverse event buttons 702 may auto-populate with adverse events that may be relevant to the current patient images 112 and the current patient contours 114 as a clinician scrolls through each patent image. A clinician may also manually add an adverse event to the table of recommended treatment results if the adverse event doesn't appear on an adverse event button 702. In some embodiments, an adverse event button may be emphasized (e.g., highlighted, flashing, or the like) in the treatment results display module 132 if the system 100 detects that a corresponding outcome of the adverse event is above a threshold limit, indicating that the adverse event may be significant and should be considered as a part of the recommended treatment results.

The plurality of adverse event removal buttons 704 allow a clinician to remove adverse events from the table of recommended treatment results. In the embodiment of FIG. 7, an adverse event removal button 704 is located next to the row of each adverse event. When selected, the entire row associated with the adverse event is removed from the table. A clinician may select to remove an adverse event if the adverse event is deemed irrelevant or is not a concern for the current patient. By removing unnecessary adverse events from the treatment results display module 132, the clinician is able to focus on the important information to determine an optimal treatment result for the current patient.

The plurality of treatment result removal buttons 706 allow a clinician to remove recommended treatment results from the table of recommended treatment results. In the embodiment of FIG. 7, a treatment result removal button 706 is located above the column of each treatment result match 138. When selected, the entire column associated with the recommended treatment result is removed from the table. A clinician may select to remove a recommended treatment result if the clinician has determined that alternate recommended treatment results are more suitable for the current patient. By removing unnecessary recommended treatment results from the treatment results display module 132, the clinician is able to more efficiently narrow down the recommended treatment results to determine an optimal treatment result for the current patient. In some embodiments, the treatment result prediction 136 may or may not be removed by a treatment result removal button 706.

The plurality of treatment result optimization buttons 708 allows a clinician to select a recommended treatment result for optimization. In the embodiment of FIG. 7, a treatment result optimization button 708 is located next to the column heading of each recommended treatment result. A clinician may select a treatment result optimization button 708 for a recommended treatment result if the clinician believes the selected recommended treatment result can be modified to achieve an optimal treatment result for the current patient. When a recommended treatment result is selected for optimization, the treatment results display module allows the clinician to define a plurality of filtering criteria, such as excluding an adverse event from the treatment result or to place a threshold limit on an outcome of an adverse event. For example, the clinician may specify a first filtering criteria to exclude "Rib Fracture" as an adverse event and a second filtering criteria to limit "Pneumonitis" to a toxicity grade of level 1 (for example, on a scale of 1-4, with 4 as the most severe). The selected recommended treatment result and the filtering criteria are input into the treatment result match navigation module 128 to identify a new set of treatment result matches 138 for the current patient. Subsequently, the treatment result match navigation module 128 sends the new set of treatment result matches to the treatment results matches 138 for display in the treatment results display module 132. The clinician may analyze the new set of recommended treatment results to determine if any of the options are optimal treatment results for the current patient. In some embodiments, if the clinician determines that one of the recommended treatment results is optimal for the current patient, the clinician may select the treatment result optimization button 708 of the recommended treatment result to export the selected recommended treatment result to the treatment delivery engine 106 to generate a patient-specific treatment delivery plan based on the selected recommended treatment result.

By simultaneously displaying the visualization module and the treatment results display module 132, a clinician may visualize the relationship between radiation delivery to the current patient contours 114 and the resulting recommended treatment results. Using the user interface module 126, the clinician is able to adjust contours and in real-time evaluate the impact on the tumor volume and the toxicity risk to the nearby anatomical structures. The clinician may go through a cycle of scrolling through each of the plurality of current patient images 112 to modify contours, add or remove adverse events from consideration, add or remove recommended treatment results from consideration, and optimize a selected treatment result until the clinician determines an optimal treatment result for the current patient. This configuration enables the clinician to make informed decisions about certain trade-offs during radiation treatment planning.

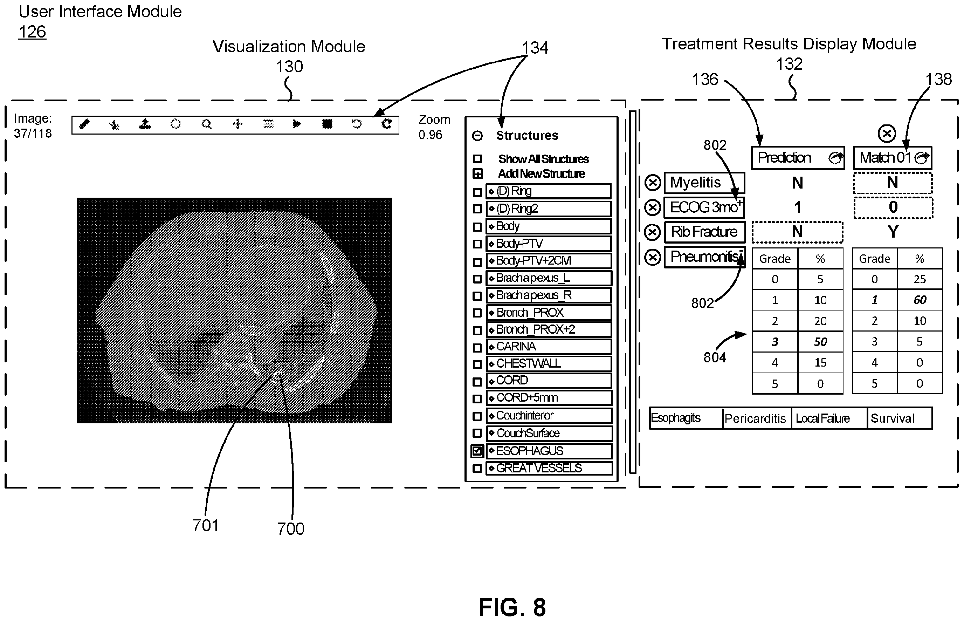

FIG. 8 illustrates an exemplary user interface for visualizing recommended treatment results, according to an additional embodiment. As described with regards to FIG. 7, each element of the table describes an outcome of an adverse event of a corresponding recommended treatment result. Each adverse event is associated with one or more of the following outcomes: a probability of level of toxicity to each identified anatomical structure, and a highest probable level of toxicity to each identified anatomical structure. In the embodiment of FIG. 7, for ease of display, only the highest probable toxicity grade may be displayed for each adverse event. In the embodiment of FIG. 8, an adverse event may include a toggle button 802 next to the row heading. Selecting the toggle button 802 causes an outcome table, such as outcome table 804, to expand for the adverse event for each recommended treatment result. As illustrated in FIG. 8, the outcome table 804 for "Pneumonitis" displays the toxicity grades and the corresponding probability for each toxicity grade. The toxicity grade with the highest probable level of toxicity may be emphasized in the outcome table 804. For example, for the treatment result prediction 136, toxicity grade 3 pneumonitis has a 50% probability of occurring as a treatment result, which is higher than the probabilities of the remaining toxicity grades. Thus, as described with regards to FIG. 7, for ease of display, only toxicity grade 3 is displayed in the treatment results display module 132 when the toggle button 802 is selected to minimize the outcome table 804. In the embodiment of FIG. 8, only adverse events that have corresponding outcomes related to toxicity grades may have a toggle button 802.

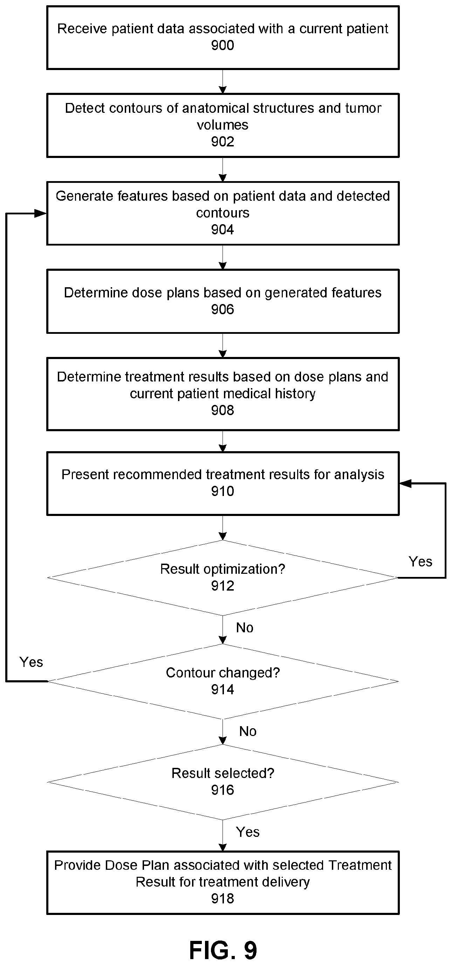

FIG. 9 illustrates a flow diagram illustrating the steps for clinician-directed radiation treatment planning, according to an embodiment. The flow diagram represents a workflow for creating a radiation treatment plan using system 100, as described with regards to FIG. 1.

As described in 900, a patient data engine receives patient data associated with a current patient. The current patient data may include patient images, patient contours, physics parameters, and prescription parameters. The current patient data is input into a treatment planning engine.

As described in 902, the treatment planning engine detects contours of anatomical structures and tumor volumes in the current patient data. These contours may be defined in each patient image by a clinician before the current patient data is imported into the system.

As described in 904, the treatment planning engine generates features associated with the current patient based on the current patient data and the detected contours. The generated features can be compared to similar generated features of prior patients to identify prior patients that are similar to the current patient.

As described in 906, the treatment planning engine determines a plurality of dose plans. The dose plans include a dose plan prediction and a plurality of dose plan matches. To determine the dose plan prediction, the treatment planning engine inputs the generated features of the current patient into a dose plan prediction model, which generates the dose plan prediction. To determine the plurality of dose plan matches, the treatment planning engine uses the dose plan prediction and the generated features of the current patient. The treatment planning engine compares the generated features of the current patient with generated features of prior patients to identify previously-administered dose plans of prior patients that are similar to the current patient. The treatment planning engine then determines which of the previously-administered dose plans are within a threshold difference of the dose plan prediction. Those within the threshold difference are the dose plan matches. The plurality of dose plan matches and the dose plan prediction comprise a set of dose plans.

As described in 908, the treatment planning engine determines a plurality of treatment results based on dose plans and current patient medical history. The treatment results include a treatment result prediction and a plurality of treatment result matches. To determine the treatment result prediction, the treatment planning engine inputs the dose plans and the current patient medical history into a treatment results prediction model, which generates the treatment result prediction. To determine the plurality of treatment result matches, the treatment planning engine uses the treatment result prediction, the dose plans, and the current patient medical history. The treatment planning engine compares the dose plans with previously-administered dose plans of prior patients to identify treatment results of previously-administered dose plans of prior patients. The treatment planning engine then determines which of the identified treatment results are within a threshold difference of the treatment result prediction. Those within the threshold difference are the treatment result matches. The plurality of treatment result matches and the treatment result prediction comprise a set of recommended treatment results.

As described in 910, the treatment planning engine presents the recommended treatment results for analysis by a clinician. The set of recommended treatment results and the current patient images and the current patient contours on which the recommended treatment results are based are simultaneously displayed. By simultaneously displaying them, the clinician may visualize the relationship between radiation delivery to the current patient contours and the resulting recommended treatment results. The clinician is able to adjust contours and in real-time evaluate the impact on the tumor volume and the toxicity risk to the nearby anatomical structures. This allows the clinician to make informed decisions about certain trade-offs during radiation treatment planning for the current patient. The clinician may modify the current patient contours to generate a new set of recommended treatment results, or the clinician may select a recommended treatment result for optimization, allowing the clinician to create a treatment plan with optimal treatment results.

As described in 912, a clinician may select a recommended treatment result for optimization. If a treatment result is selected, the clinician may define a plurality of filtering criteria. The treatment planning engine accesses a data store to identify a new set of recommended treatment results based on the selected treatment result and the filtering criteria. The new set of recommended treatment results is presented for analysis by the clinician, as discussed in 910.

As described in 914, a clinician is able to modify the current patient contours. If the treatment planning engine detects that a contour is modified, a new set of current patient contours is captured. As a result, 904-914 may be repeated.

As described in 916, a clinician may select a recommended treatment result as an optimal treatment result for the current patient. The selected treatment result is sent to a treatment delivery engine to create a radiation dose treatment plan that will produce the selected treatment result.

As described in 918, the treatment delivery engine provides a radiation dose treatment plan associated with the selected treatment result to a clinician for treatment delivery to the current patient. The invention is defined in the following claims:

## Claims

1. A computer-implemented method for creating a radiation treatment plan for a given patient, the method comprising:
receiving imaging data that includes a representation of an interior of a patient who is to receive radiation treatment (900);
repeatedly performing a treatment planning process comprising:
detecting at least one contour identifying one or more anatomical structures and at least one tumor volume represented by the imaging data (902);
computing a set of dose treatment plans for the patient based on the at least one contour and a set of prior patient dose treatment plans (906);
computing one or more recommended treatment results for the patient based on the at least one contour, a set of prior patient treatment results, and the set of prior patient dose treatment plans (908); and
presenting in a user interface the set of recommended treatment results to a clinician for evaluation (910);
**characterized in that** the method further comprises:
receiving a user input via the user interface providing at least one modified contour;
presenting in the user interface, in real-time as the user input is received, a modified set of recommended treatment results generated based on the at least one modified contour, wherein the treatment planning process is repeated until a selection is made by the clinician of one of the modified set of recommended treatment results;
receiving the selection from the clinician of said recommended treatment result; and
in response to a selection from the clinician, providing the selected recommended treatment result to a system for generating a treatment plan for the patient that achieves the selected recommended treatment result.

2. The method of claim 1,
wherein the generated treatment plan comprises a dose treatment plan associated with the selected recommended treatment result for administering to the given patient.

3. A computer system for creating a radiation treatment plan for a given patient, the system comprising:
a non-transitory computer-readable storage medium storing executable computer program instructions comprising instructions for:
receiving imaging data that includes a representation of an interior of a patient who is to receive radiation treatment;
repeatedly performing a treatment planning process comprising:
detecting at least one contour identifying one or more anatomical structures and at least one tumor volume represented by the imaging data;
computing a set of dose treatment plans for the patient based on the at least one contour and a set of prior patient dose treatment plans;
computing one or more recommended treatment results for the patient based on the at least one contour, a set of prior patient treatment results, and the set of prior patient dose treatment plans;
presenting in a user interface the set of recommended treatment results to a clinician for evaluation; and
a processor for executing the computer program instructions;
wherein the system is **characterized in that** the non-transitory computer-readable storage medium storing executable computer program instructions further comprises instructions for:
receiving a user input via the user interface providing at least one modified contour;
presenting in the user interface, in real-time as the user input is received, a modified set of recommended treatment results generated based on the at least one modified contour, wherein the treatment planning process is repeated until a selection is made by the clinician of one of the modified set of recommended treatment results; receiving the selection from the clinician of said recommended treatment result; and
in response to the selection from the clinician, providing the selected recommended treatment result to a system for generating a treatment plan for the patient that achieves the selected recommended treatment result.

4. The computer system of claim 3, wherein the generated treatment plan comprises a dose treatment plan associated with the selected recommended treatment result for administering to the given patient.

5. The computer system of any of claims 3 to 4, wherein a treatment result represents a set of one or more adverse events, wherein each adverse event is associated with one or more of the following outcomes: a probability of level of toxicity to each identified anatomical structure, and a highest probable level of toxicity to each identified anatomical structure, and/or wherein a treatment result indicates one or more of the following: a probability of tumor recurrence, a type of tumor recurrence such as local recurrence or distant recurrence, and a probable length of time before tumor recurrence.

6. The computer system of any of claims 3 to 5, wherein a first treatment result of the set of recommended treatment results is a predicted treatment result that is generated using a treatment result model based on prior patient dose treatment plans and treatment results associated with the prior patient dose treatment plans.

7. The computer system of claim 6, wherein the treatment result model is further based on medical history of the prior patients associated with the dose treatment plans.

8. The computer system of claim 7, wherein computing the set of recommended treatment results comprises:
computing the first treatment result using the treatment result model; and
determining a set of prior patient treatment results that are within a threshold difference from the first treatment result.

9. The computer system of any of claims 3 to 8, wherein the computer program instructions further comprise instructions for:
receiving a selection from the clinician of one of the recommended treatment results for optimization, wherein the optimization of the selected recommended treatment result comprises applying one or more filtering criteria defined by the clinician;
determining a new set of prior patient treatment results that are within a threshold difference from the selected recommended treatment result and based on the one or more filtering criteria; and
presenting in the user interface the new set of prior patient treatment results to a clinician for evaluation.

10. The computer system of claim 9, wherein the filtering criteria comprise one or more of the following: an exclusion of an adverse event from the treatment result or a threshold limit on an outcome of an adverse event.

11. The computer system of any of claims 3 to 10, wherein detecting the at least one contour comprises: determining that a previously-detected contour has been modified or detecting a new contour identifying one or more anatomical structures and at least one tumor volume represented by the imaging data.

12. The computer system of any of claims 3 to 11, wherein the user interface enables the clinician to manually define one or more contours, remove a previously-detected contour, or modify a previously-detected contour on a plurality of selected anatomical structures on a visual representation of the imaging data, and/or wherein the user interface graphically presents:
a plurality of adverse events that may result from the set of dose treatment plans;
a predicted treatment result for the given patient that provides a predicted outcome for each adverse event; and
one or more prior patient treatment results, wherein each prior patient treatment result provides an outcome for each adverse event, the provided outcome a result of the prior patient dose treatment plan associated with the prior patient treatment result.

13. The computer system of claim 12, wherein the computer program instructions further comprise instructions for:
detecting an addition, removal, or modification of at least one contour in the user interface results in computing a new set of recommended treatment results that are displayed in the user interface; and/or
wherein the user interface comprises a list of additional adverse events that may be selected for display in the user interface, such than an outcome of each selected adverse event is displayed in the user interface; and/or
wherein the user interface is configured to allow a clinician to remove one or more of the adverse events from displaying in the user interface.

## Patentansprüche

1. Computerimplementiertes Verfahren zum Erstellen eines Strahlungsbehandlungsplans für einen gegebenen Patienten, wobei das Verfahren Folgendes umfasst:
Empfangen von Bilddaten, die eine Darstellung eines Inneren eines Patienten, der Strahlungsbehandlung (900) erhalten soll, umfassen;
wiederholtes Ausführen eines Behandlungsplanungsprozesses, der Folgendes umfasst:
Erfassen mindestens einer von den Bilddaten (902) dargestellten Kontur, die eine oder mehrere anatomische Strukturen und mindestens ein Tumorvolumen identifiziert;
Berechnen eines Satzes von Dosisbehandlungsplänen für den Patienten basierend auf der mindestens einen Kontur und einem Satz von Dosisbehandlungsplänen (906) früherer Patienten;
Berechnen eines oder mehrerer empfohlener Behandlungsergebnisse für den Patienten basierend auf der mindestens einen Kontur, einem Satz von Behandlungsergebnissen früherer Patienten und dem Satz von Dosisbehandlungsplänen (908) früherer Patienten; und
Präsentieren, in einer Benutzeroberfläche, des Satzes von empfohlenen Behandlungsergebnissen für einen Mediziner zur Auswertung (910);
**dadurch gekennzeichnet, dass** das Verfahren ferner Folgendes beinhaltet:
Empfangen einer Benutzereingabe über die Benutzeroberfläche, die mindestens eine abgeänderte Kontur bereitstellt;
Präsentieren, in der Benutzeroberfläche, in Echtzeit, während die Benutzereingabe empfangen wird, eines basierend auf der mindestens einen abgeänderten Kontur erzeugten abgeänderten Satzes von empfohlenen Behandlungsergebnissen, wobei der Behandlungsplanungsprozess wiederholt wird, bis von dem Mediziner eine Auswahl von einem aus dem abgeänderten Satz von empfohlenen Behandlungsergebnissen getroffen wird;
Empfangen der Auswahl des Mediziners des empfohlenen Behandlungsergebnisses; und
als Reaktion auf eine Auswahl des Mediziners, Bereitstellen des ausgewählten empfohlenen Behandlungsergebnisses für ein System zum Erzeugen eines Behandlungsplans für den Patienten, der das ausgewählte empfohlene Behandlungsergebnis erreicht.

2. Verfahren nach Anspruch 1, wobei der erzeugte Behandlungsplan einen mit dem ausgewählten empfohlenen Behandlungsergebnis assoziierten Dosisbehandlungsplan zum Verabreichen an den gegebenen Patienten umfasst.

3. Computersystem zum Erstellen eines Strahlungsbehandlungsplans für einen gegebenen Patienten, wobei das System Folgendes umfasst:
ein nichtflüchtiges computerlesbares Speichermedium, das ausführbare Computerprogrammanweisungen speichert, die Anweisungen zu Folgendem umfassen:
Empfangen von Bilddaten, die eine Darstellung eines Inneren eines Patienten, der Strahlungsbehandlung erhalten soll, umfassen;
wiederholtes Ausführen eines Behandlungsplanungsprozesses, der Folgendes umfasst:
Erfassen mindestens einer von den Bilddaten dargestellten Kontur, die eine oder mehrere anatomische Strukturen und mindestens ein Tumorvolumen identifiziert;
Berechnen eines Satzes von Dosisbehandlungsplänen für den Patienten basierend auf der mindestens einen Kontur und einem Satz von Dosisbehandlungsplänen früherer Patienten;
Berechnen eines oder mehrerer empfohlener Behandlungsergebnisse für den Patienten basierend auf der mindestens einen Kontur, einem Satz von Behandlungsergebnissen früherer Patienten und dem Satz von Dosisbehandlungsplänen früherer Patienten;
Präsentieren, in einer Benutzeroberfläche, des Satzes von empfohlenen Behandlungsergebnissen für einen Mediziner zur Auswertung; und
einen Prozessor zum Ausführen der Computerprogrammanweisungen;
wobei das System **dadurch gekennzeichnet ist, dass** das ausführbare Computerprogrammanweisungen speichernte nichtflüchtige computerlesbare Speichermedium ferner Anweisungen zu Folgendem umfasst:
Empfangen einer Benutzereingabe über die Benutzeroberfläche, die mindestens eine abgeänderte Kontur bereitstellt;
Präsentieren, in der Benutzeroberfläche, in Echtzeit, während die Benutzereingabe empfangen wird, eines basierend auf der mindestens einen abgeänderten Kontur erzeugten abgeänderten Satzes von empfohlenen Behandlungsergebnissen, wobei der Behandlungsplanungsprozess wiederholt wird, bis von dem Mediziner eine Auswahl von einem aus dem abgeänderten Satz von empfohlenen Behandlungsergebnissen getroffen wird;
Empfangen der Auswahl des Mediziners des empfohlenen Behandlungsergebnisses; und
als Reaktion auf die Auswahl des Mediziners, Bereitstellen des ausgewählten empfohlenen Behandlungsergebnisses für ein System zum Erzeugen eines Behandlungsplans für den Patienten, der das ausgewählte empfohlene Behandlungsergebnis erreicht.

4. Computersystem nach Anspruch 3, wobei der erzeugte Behandlungsplan einen mit dem ausgewählten empfohlenen Behandlungsergebnis assoziierten Dosisbehandlungsplan zum Verabreichen an den gegebenen Patienten umfasst.

5. Computersystem nach einem der Ansprüche 3 bis 4, wobei ein Behandlungsergebnis einen Satz von einem oder mehreren unerwünschten Ereignissen darstellt, wobei jedes unerwünschte Ereignis mit einem oder mehreren der folgenden Ausgänge assoziiert ist: einer Wahrscheinlichkeit eines Niveaus der Toxizität für jede identifizierte anatomische Struktur und ein höchstes wahrscheinliches Niveau der Toxizität für jede identifizierte anatomische Struktur, und/oder wobei ein Behandlungsergebnis eines oder mehrere der Folgenden angibt: eine Wahrscheinlichkeit des Wiederauftretens des Tumors, einer Art des Wiederauftretens des Tumors, wie etwa lokales Wiederauftreten oder entferntes Wiederauftreten, und eine Wahrscheinliche Zeitspanne vor dem Wiederauftreten des Tumors.

6. Computersystem nach einem der Ansprüche 3 bis 5, wobei ein erstes Behandlungsergebnis des Satzes von empfohlenen Behandlungsergebnissen ein vorhergesagtes Behandlungsergebnis ist, das unter Verwendung eines Behandlungsergebnismodells erzeugt wird, das auf Dosisbehandlungsplänen früherer Patienten und mit den Dosisbehandlungsplänen früherer Patienten assoziierten Behandlungsergebnissen basiert.

7. Computersystem nach Anspruch 6, wobei das Behandlungsergebnismodell ferner auf der Krankengeschichte der mit den Dosisbehandlungsplänen assoziierten früheren Patienten basiert.

8. Computersystem nach Anspruch 7, wobei das Berechnen des Satzes von empfohlenen Behandlungsergebnissen Folgendes umfasst:
Berechnen des ersten Behandlungsergebnisses unter Verwendung des Behandlungsergebnismodells; und
Bestimmen eines Satzes von Behandlungsergebnissen früherer Patienten, die innerhalb einer Schwellendifferenz von dem ersten Behandlungsergebnis liegen.

9. Computersystem nach einem der Ansprüche 3 bis 8, wobei die Computerprogrammanweisungen ferner Anweisungen zu Folgendem umfassen:
Empfangen einer Auswahl des Mediziners von einem der empfohlenen Behandlungsergebnisse zur Optimierung, wobei die Optimierung des ausgewählten empfohlenen Behandlungsergebnisses das Anwenden von einem oder mehreren von dem Mediziner definierten Filterkriterien umfasst;
Bestimmen eines neuen Satzes von Behandlungsergebnissen früherer Patienten, die innerhalb einer Schwellendifferenz von dem ausgewählten empfohlenen Behandlungsergebnis liegen und auf dem einen oder den mehreren Filterkriterien basieren; und
Präsentieren, in der Benutzeroberfläche, des neuen Satzes von Behandlungsergebnissen früherer Patienten für einen Mediziner zur Auswertung.

10. Computersystem nach Anspruch 9, wobei die Filterkriterien eines oder mehrere der Folgenden umfassen: ein Ausschließen eines unerwünschten Ereignisses aus dem Behandlungsergebnis oder einen Schwellenwert für einen Ausgang eines unerwünschten Ereignisses.

11. Computersystem nach einem der Ansprüche 3 bis 10, wobei das Erfassen der mindestens einen Kontur Folgendes umfasst: Bestimmen, dass eine zuvor erfasste Kontur abgeändert wurde, oder Erfassen einer neuen von den Bilddaten dargestellten Kontur, die eine oder mehrere anatomische Strukturen und mindestens ein Tumorvolumen identifiziert.

12. Computersystem nach einem der Ansprüche 3 bis 11, wobei die Benutzeroberfläche es dem Mediziner ermöglicht, eine oder mehrere Konturen manuell zu definieren, eine zuvor erfasste Kontur zu entfernen oder eine zuvor erfasste Kontur auf einer Vielzahl von ausgewählten anatomischen Strukturen auf einer visuellen Darstellung der Bilddaten abzuändern, und/oder wobei die Benutzeroberfläche Folgendes grafisch präsentiert:
eine Vielzahl von unerwünschten Ereignissen, die aus dem Satz von Dosisbehandlungsplänen resultieren können;
ein vorhergesagtes Behandlungsergebnis für den gegebenen Patienten, das einen vorhergesagten Ausgang für jedes unerwünschte Ereignis bereitstellt; und
ein oder mehrere Behandlungsergebnisse früherer Patienten, wobei jedes Behandlungsergebnis früherer Patienten einen Ausgang für jedes unerwünschte Ereignis bereitstellt, wobei der bereitgestellte Ausgang ein Ergebnis des mit dem Behandlungsergebnis früherer Patienten assoziierten Dosisbehandlungsplans früherer Patienten ist.

13. Computersystem nach Anspruch 12, wobei die Computerprogrammanweisungen ferner Anweisungen zu Folgendem umfassen:
Erfassen eines Ergänzens, eines Entfernens oder eines Abänderns mindestens einer Kontur in der Benutzeroberfläche, was zum Berechnen eines neuen Satzes von empfohlenen Behandlungsergebnissen, die in der Benutzeroberfläche angezeigt werden, führt; und/oder
wobei die Benutzeroberfläche eine Liste von zusätzlichen unerwünschten Ereignissen umfasst, die zur Anzeige in der Benutzeroberfläche ausgewählt werden können, sodass ein Ausgang jedes ausgewählten unerwünschten Ereignisses in der Benutzeroberfläche angezeigt wird; und/oder
wobei die Benutzeroberfläche dazu konfiguriert ist, es einem Mediziner zu ermöglichen, eines oder mehrere der unerwünschten Ereignisse aus der Anzeige in der Benutzeroberfläche zu entfernen.

## Revendications

1. Procédé mis en œuvre par ordinateur pour la création d'un plan de radiothérapie pour un patient donné, le procédé comprenant :
le fait de recevoir des données d'imagerie qui incluent une représentation d'un intérieur d'un patient qui doit recevoir une radiothérapie (900) ;
le fait d'exécuter de manière répétée un processus de planification de traitement comprenant :
le fait de détecter au moins un contour identifiant une ou plusieurs structures anatomiques et au moins un volume tumoral représenté par les données d'imagerie (902) ;
le fait de calculer un ensemble de plans de traitement de dose pour le patient sur la base de l'au moins un contour et d'un ensemble de plans de traitement de dose de patients antérieurs (906) ;
le fait de calculer un ou plusieurs résultats de traitement recommandé pour le patient sur la base de l'au moins un contour, d'un ensemble de résultats de traitement de patients antérieurs, et de l'ensemble de plans de traitement de dose de patients antérieurs (908) ; et
le fait de présenter dans une interface utilisateur l'ensemble des résultats de traitement recommandé à un clinicien pour évaluation (910) ;
**caractérisé en ce que** le procédé comprend en outre :
le fait de recevoir une entrée utilisateur par le biais de l'interface utilisateur fournissant au moins un contour modifié ;
le fait de présenter dans l'interface utilisateur, en temps réel à mesure que l'entrée utilisateur est reçue, un ensemble modifié de résultats de traitement recommandé générés sur la base de l'au moins un contour modifié, où le processus de planification de traitement est répété jusqu'à ce qu'une sélection soit faite par le clinicien d'un résultat de l'ensemble modifié de résultats de traitement recommandé ;
le fait de recevoir la sélection du clinicien dudit résultat de traitement recommandé ; et
en réponse à une sélection du clinicien, le fait de fournir le résultat de traitement recommandé sélectionné à un système pour la génération d'un plan de traitement pour le patient qui atteint le résultat de traitement recommandé sélectionné.

2. Procédé selon la revendication 1, où le plan de traitement généré comprend un plan de traitement de dose associé au résultat de traitement recommandé sélectionné pour l'administration au patient donné.

3. Système informatique pour la création d'un plan de radiothérapie pour un patient donné, le système comprenant :
un support de stockage lisible par ordinateur non transitoire stockant des instructions de programme informatique exécutables comprenant des instructions pour :
recevoir des données d'imagerie qui incluent une représentation d'un intérieur d'un patient qui doit recevoir une radiothérapie ;
exécuter de manière répétée un processus de planification de traitement comprenant :
le fait de détecter au moins un contour identifiant une ou plusieurs structures anatomiques et au moins un volume tumoral représenté par les données d'imagerie ;
le fait de calculer un ensemble de plans de traitement de dose pour le patient sur la base de l'au moins un contour et d'un ensemble de plans de traitement de dose de patients antérieurs ;
le fait de calculer un ou plusieurs résultats de traitement recommandé pour le patient sur la base de l'au moins un contour, d'un ensemble de résultats de traitement de patients antérieurs, et de l'ensemble de plans de traitement de dose de patients antérieurs ;
le fait de présenter dans une interface utilisateur l'ensemble des résultats de traitement recommandé à un clinicien pour évaluation ; et
un processeur pour l'exécution des instructions de programme informatique ;
où le système est **caractérisé en ce que** le support de stockage lisible par ordinateur non transitoire stockant des instructions de programme informatique exécutables comprend en outre des instructions pour :
recevoir une entrée utilisateur par le biais de l'interface utilisateur fournissant au moins un contour modifié ;
présenter dans l'interface utilisateur, en temps réel à mesure que l'entrée utilisateur est reçue, un ensemble modifié de résultats de traitement recommandé générés sur la base de l'au moins un contour modifié, où le processus de planification de traitement est répété jusqu'à ce qu'une sélection soit faite par le clinicien d'un résultat de l'ensemble modifié de résultats de traitement recommandé ;
recevoir la sélection du clinicien dudit résultat de traitement recommandé ; et
en réponse à la sélection du clinicien, fournir le résultat de traitement recommandé sélectionné à un système pour la génération d'un plan de traitement pour le patient qui atteint le résultat de traitement recommandé sélectionné.

4. Système informatique selon la revendication 3, où le plan de traitement généré comprend un plan de traitement de dose associé au résultat de traitement recommandé sélectionné pour l'administration au patient donné.

5. Système informatique selon l'une quelconque des revendications 3 à 4, où un résultat de traitement représente un ensemble d'un ou plusieurs événements indésirables, où chaque événement indésirable est associé à une ou plusieurs des issues suivantes : une probabilité de niveau de toxicité pour chaque structure anatomique identifiée, et un niveau de toxicité le plus probable pour chaque structure anatomique identifiée, et/ou où un résultat de traitement indique un ou plusieurs des éléments suivants : une probabilité de récidive tumorale, un type de récidive tumorale tel qu'une récidive locale ou une récidive distante, et une durée probable avant la récidive tumorale.

6. Système informatique selon l'une quelconque des revendications 3 à 5, où un premier résultat de traitement de l'ensemble de résultats de traitement recommandé est un résultat de traitement prédit qui est généré à l'aide d'un modèle de résultat de traitement sur la base de plans de traitement de dose de patients antérieurs et de résultats de traitement associés aux plans de traitement de dose de patients antérieurs.

7. Système informatique selon la revendication 6, où le modèle de résultat de traitement est en outre basé sur l'historique médical des patients antérieurs associés aux plans de traitement de dose.

8. Système informatique selon la revendication 7, où le fait de calculer l'ensemble des résultats de traitement recommandé comprend :
le fait de calculer le premier résultat de traitement à l'aide du modèle de résultat de traitement ; et
le fait de déterminer un ensemble de résultats de traitement de patients antérieurs qui sont compris dans les limites d'une différence de seuil par rapport au premier résultat de traitement.

9. Système informatique selon l'une quelconque des revendications 3 à 8, où les instructions de programme informatique comprennent en outre des instructions pour :
recevoir une sélection du clinicien de l'un des résultats de traitement recommandé pour optimisation, où l'optimisation du résultat de traitement recommandé sélectionné comprend l'application d'un ou plusieurs critères de filtrage définis par le clinicien ;
déterminer un nouvel ensemble de résultats de traitement de patients antérieurs qui sont compris dans les limites d'une différence de seuil par rapport au résultat de traitement recommandé sélectionné et sur la base des un ou plusieurs critères de filtrage ; et
présenter dans l'interface utilisateur le nouvel ensemble de résultats de traitement de patients antérieurs à un clinicien pour évaluation.

10. Système informatique selon la revendication 9, où les critères de filtrage comprennent un ou plusieurs des éléments suivants : une exclusion d'un événement indésirable du résultat de traitement ou une limite de seuil sur une issue d'un événement indésirable.

11. Système informatique selon l'une quelconque des revendications 3 à 10, où le fait de détecter l'au moins un contour comprend : le fait de déterminer qu'un contour précédemment détecté a été modifié ou le fait de détecter un nouveau contour identifiant une ou plusieurs structures anatomiques et au moins un volume tumoral représenté par les données d'imagerie.

12. Système informatique selon l'une quelconque des revendications 3 à 11, où l'interface utilisateur permet au clinicien de définir manuellement un ou plusieurs contours, de supprimer un contour précédemment détecté, ou de modifier un contour précédemment détecté sur une pluralité de structures anatomiques sélectionnées sur une représentation visuelle des données d'imagerie, et/ou où l'interface utilisateur présente graphiquement :
une pluralité d'événements indésirables qui peuvent résulter de l'ensemble des plans de traitement de dose ;
un résultat de traitement prédit pour le patient donné qui fournit une issue prédite pour chaque événement indésirable ; et
un ou plusieurs résultats de traitement de patients antérieurs, où chaque résultat de traitement de patients antérieurs fournit une issue pour chaque événement indésirable, l'issue fournie étant un résultat du plan de traitement de dose de patients antérieurs associé au résultat de traitement de patients antérieurs.

13. Système informatique selon la revendication 12, où les instructions de programme informatique comprennent en outre des instructions pour :
détecter un ajout, une suppression, ou une modification d'au moins un contour dans l'interface utilisateur ce qui entraîne le fait de calculer un nouvel ensemble de résultats de traitement recommandé qui sont affichés dans l'interface utilisateur ; et/ou
où l'interface utilisateur comprend une liste d'événements indésirables supplémentaires qui peuvent être sélectionnés pour un affichage dans l'interface utilisateur, de sorte qu'une issue de chaque événement indésirable sélectionné est affichée dans l'interface utilisateur ; et/ou
où l'interface utilisateur est configurée pour permettre à un clinicien de supprimer un ou plusieurs des événements indésirables de l'affichage dans l'interface utilisateur.
